# EUROPÄISCHE PATENTSCHRIFT

(11) **EP 0 563 527 B1**
(45) Veröffentlichungstag und Bekanntmachung des Hinweises auf die Patenterteilung: **13.03.1996**
(21) Anmeldenummer: 93101279.3
(22) Anmeldetag: 28.01.1993
(51) Int. Cl.: C12N 15/77, C12N 15/11, C12P 19/34

(54) **Verfahren zum Auffinden von Insertionselementen (IS-Elemente) oder Transposonen**
Process for the detection of insertion elements or transposons
Procédé de mise en évidence d'éléments d'insertion ou de transposons

(30) Priorität: 19.03.1992 DE 4208785
(43) Veröffentlichungstag der Anmeldung: 06.10.1993
(73) Patentinhaber: Degussa Aktiengesellschaft, D-60311 Frankfurt (DE)
(72) Erfinder: Schäfer, Andreas, W-4800 Bielefeld 1 (DE); Seep-Feldhaus, Anna-Hildegard, W-4800 Bielefeld 14 (DE); Jäger, Wolfgang, W-4800 Bielefeld 1 (DE); Kalinowski, Jörn, Dr., W-4800 Bielefeld 1 (DE); Wohlleben, Wolfgang, Dr., W-4800 Bielefeld 1 (DE); Pühler, Alfred, Prof. Dr., W-4800 Bielefeld 15 (DE)

(56) Entgegenhaltungen:
- EP-A- 0 252 558
- EP-A- 0 372 230
- EP-A- 0 445 385
- WO-A-91/00913
- APPLICATIONS MANUAL 1989, Seiten 1 - 3, BOEHRINGER MANNHEIM GMBH BIOCHEMICA: 'DNA labeling and detection nonradioactive'
- JOURNAL OF BACTERIOLOGY, Bd. 164, 1985, Seiten 918 - 921; P. GAY ET AL.; 'Positive selection procedure for entrapment of insertion sequence elements in gram-negative bacteria'
- JOURNAL OF BACTERIOLOGY, Bd. 174, 1992, Seiten 5462 - 5465; W. JAEGER ET AL.; 'Expression of the Bacillus subtilis sacB gene leads to sucrose sensitivity in the gram-positive bacterium Corynebacterium glutanicum but not in Streptomyces lividans'

## Beschreibung

Die Erfindung betrifft ein Verfahren zum Auffinden von Insertionselementen (IS-Elemente) oder Transposonen in corvneformen Bakterien, ein dazu geeignetes positives Selektionssystem, die so gefundenen IS-Elemente und ihre Verwendung.

Insertionselemente (IS-Elemente) sind DNA-Bereiche von etwa 0,6 bis 1,8 Kilobasen (kb) Länge, die in prokaryontischen Genomen innerhalb eines Replikons oder von einem Replikon zu einem anderen springen (transponieren) können (Craig & Kleckner 1987, in Neidhardt et al. Escherichia coli and Salmonella typhimurium, Cellular and Molecular Biology, pp. 1054-1074, ASM Press, Washington, DC). Dabei kann es entweder zur konservativen Transposition kommen, d. h. ein Element wechselt seinen Platz, oder es kommt zur replikativen Transposition, wobei nur eine Kopie des Elements am neuen Insertionsort integriert, während das Original am alten Platz verbleibt. Bei der replikativen Transposition kann es zur Verschmelzung des Donor- und des Akzeptormoleküls kommen (Replikonfusion). Dieses Zwischenstadium der Transposition kann dann durch Rekombination der an den Fusionspunkten liegenden Kopien der IS-Elemente wieder aufgelöst werden. Bei geeigneter Selektion auf die Replikonfusion kann diese aber erhalten werden. IS-Elemente selbst sind im Gegensatz zu den nahe verwandten Transposonen nicht mit einer selektionierbaren Markierung ausgestattet.

IS-Elemente kodieren meistens nur für ein einziges Genprodukt, die sogenannte Transposase. Es handelt sich hierbei um ein Rekombinationsprotein, das von einem oder zwei offenen Leserastern des Insertionselements abgelesen wird und das die Transposition durch die sogenannte illegitime. d. h. vom Rekombinationssystem des Wirtsorganismus unabhängige Rekombination an den invers repetitiven Enden des Elements durchführt.

Bei der Transposition von Insertionselementen in ein bakterielles Gen wird dieses gewöhnlich zerstört, also eine Mutation erzeugt (Craig & Kleckner 1987 s.o.).

Daneben kann es durch polare Effekte, das Unterbrechen der Transkription eines Operons durch die Integration in ein vorderes Gen, zur Abschaltung weiter hinten liegender Gene kommen.

Endogene Insertionselemente können zur genetischen Instabilität eines natürlichen oder rekombinanten Mikroorganismus beitragen. Dabei werden neben Insertionen von IS-Elementen auch Deletionen angrenzender Bereich oder andere Umordnungen von DNA erzeugt. Weiterhin ist bekannt, daß Insertionselemente die Stabilität von Plasmiden besonders unter Produktionsbedingungen negativ beeinflussen können (Kumar et al. Trends Biotech. 9:279-284, 1991).

Insertionselemente wurden bisher schon in einer Anzahl verschiedener Bakteriengattungen nachgewiesen. Bei den Gram-positiven Bakterien sind Insertionselemente vor allem aus den Gattungen Bacillus, Staphylococcus, Streptococcus, Lactobacillus und Streptomyces bekannt.

Insertionselemente aus coryneformen Bakterien, insbesondere Aminosäuren produzierenden, sind bisher noch nicht beschrieben worden.

Aufgabe der Erfindung ist es, ein Verfahren zum Auffinden von Insertionselementen in coryneformen Bakterien und das dazu gehörige positive Selektionssystem zur Verfügung zu stellen.

Gegenstand der Erfindung ist ein Verfahren zum Auffinden von Insertionselementen (IS-Elementen) und Transposonen in coryneformen Bakterien bzw. die Untersuchung dieser Bakterien auf die Anwesenheit derartiger Elemente das umfaßt:
- 1.1.: die Konstruktion eines aus einem E.coli Mobilisatorstamm mobilisierbaren, nicht selbsttransferierbaren Vektors, zusammengesetzt aus
- 1.1.1: einem DNA-Segment, enthaltend ein in E.coli funktionelles Replikon,
- 1.1.2: einem zweiten DNA-Segment, enthaltend das für die Mobilisierungsfunktion codierende DNA-Fragment (Mob-site enthaltend den oriT).
- 1.1.3: einem dritten DNA-Segment, das in Gram-positiven Bakterien homolog rekombiniert und/oder ein in coryneformen Bakterien funktonelles Replikon enthält,
- 1.1.4: einem das sacB-Gen enthaltenden DNA-Segment aus Bacillus subtilis,
- 1.2.: Übertragung dieses Vektors durch konjugativen Transfer in die coryneformen Rezipientenstämme,
- 1.3.: Anzucht der den Vektor enthaltenden Transkonjuganten in einem ∼ 10 % Sucrose-haltigen Nährmedium,
- 1.4.: Lyse der Sucrose-resistenten Klone, Spaltung der Plasmide mit Restriktionsendonucleasen und Analyse der Fragmente.

Die Konstruktion der geeigneten Vektoren -jedoch nicht deren Wirkung als Fangvektoren- wird im Prinzip ebenso wie das Verfahren zum konjugativen Transfer in der DE-OS 38 41453 beschrieben.

Dieses ist dadurch gekennzeichnet, daß man bevorzugt restriktionsdefekte Zellen eines Gram-positiven Bakteriums herstellt und diese nach an sich bekannten Kreuzungsverfahren mit einem den mobilisierbaren Vektor tragenden E.coli Mobilisatorstamm mischt. Das Fehlen eines funktionsfähigen Restriktionssystems bzw. der Hitzeschock erleichtert den Transfer, es ist aber nicht eine notwendige Voraussetzung dafür.

Während sich der Donor bevorzugt in der logatihmischen Wachstumsphase befindet, hat sich für den Zustand des Rezipienten die stationäre Wachstumsphase als günstig erwiesen.

Donor- und Rezipientenzellen werden im allgemeinen im Verhältnis 1 : 1 bis 1 : 10, bevorzugt 1 : 1 bis 1 : 6. eingesetzt.

Die geeigneten mobilisierbaren Vektoren sind nicht selbsttransferierbar.

Unter Punkt 1.1 verstehen sich allgemein alle in E.coli-Stämmen selbständig replizierden Plasmide (Vektoren), die sich nach dem Stand der Technik als für gentechnologische Anwendungen nützlich erwiesen haben.

Beispiele solcher E.coli Vektoren sind pMB9, pBR322, pBR325, pKB111, pUC8, pUC9, pACYC184, pACYC177, pSC101.

Übliche E.coli Vektoren wie pBR325 (Bolivar, F. et al., Gene 2, 95, (1977) oder pACYC184 (Chang, A.C.Y. und Cohen, S.N., J. Bact. 134, 1141 (1978) sind weder selbsttransferierbar noch ausreichend mobilisierbar.

Diese und andere Vektoren, die nur in Bakterienstämmen der E.coli-Gruppe replizieren, werden durch Insertion der Mob-site eines Plasmids mit weitem Wirtsbereich in Gram-negativen Bakterien modifiziert.

Bevorzugt wird das Plasmid RP4 für diese Zwecke verwendet. Derartige Vektoren, die ein ∼ 1,9 kb großes Fragment (Mob-site) von RP4 tragen, lassen sich in dem erfindungsgemäßen Verfahren vorteilhaft einsetzen.

Als Mobilisatorstämme geeignet sind modifizierte E.coli-Stämme, die ein Plasmid im Chromosom integriert oder frei vorliegend enthalten, das in der Lage ist, die zur Mobilisierung notwendigen Funktionen bereitzustellen.

Es eignen sich insbesondere Stämme, in deren Chromosom ein RP4-Derivat integriert ist, dessen Transfer-Funktion in trans auf die Mob-site der oben genannten Vektoren einwirkt.

Geeignete Vektoren und E.coli Mobilisatorstämme, wie z. B. SM-10, S68-7 und S17-1 sind aus der US-PS 4,626,504 bekannt. Der den Transfer erleichternde Restriktionsdefekt kann genetisch bedingt sein und z. B. durch mutagene Agentien (z. B. NTG: Methylnitronitrosoguanidin), erzeugt werden, er kann aber auch physiologisch bedingt sein,z. B. durch einen Hitzeschock. Als besonders effektiv hat sich die Hitzebehandlung des Rezipienten unmittelbar vor der Kreuzung erwiesen. Dabei sind intakte oder sphäroplastierte Zellen einzusetzen.

Damit gelingt es erstmals, gezielt Insertionselemente in Gram-positiven Bakterien zu finden.

Das für diesen Zweck eingesetzte positive Selektionssystem (sacB-System) zum Auffinden von Insertionselementen in coryneformen Bakterien umfaßt einen mobilisierbaren, nicht selbsttransferierbaren Vektor, der zusammengesetzt ist aus:
a) einem DNA-Segment, enthaltend ein in E.coli funktionelles Replikon,
b) einem zweiten DNA-Segment, enthaltend das für die Mobilisierungsfunktion codierende DNA-Fragment (Mob-site enthaltend den oriT),
c) einem dritten DNA-Segment, das in Gram-positiven Bakterien homolog rekombiniert und gebenenfalls ein in coryneformen Bakterien funtionelles Replikon enthält, und
d) einem das sacB-Gen enthaltenden DNA-Segment aus Bacillus subtilis.

Für die erfindungsgemäße Isolierung von Insertionssequenzen (IS-Elementen) oder Transposonen wird das sacB-Gen aus Bacillus subtilis verwendet (Gay et al., J. Bacteriol. 153:1424-1431, 1983) Das Gen kodiert für das Exoenzym Levansucrase, welches die Reaktionen Saccharosehydrolyse und Levan-Synthese katalysiert (Dedonder et al., Methods in Enzymol. 8:500-505, 1966). Die Expression von sacB in E.coli führt zum Transport des Enzyms ins Periplasma (Steinmetz et al. Mol. Gen. Genet. 191:138-144, 1983) und ist für E.coli und andere Gram-negative Bakterien letal auf Medien mit über 5 % Sucrose (Gay et al., J.Bacteriol. 164:918-921).

Es wurde nun gefunden, daß die Expression des intakten sacB-Genes auch in Gram-positiven Bakterien, wie z. B. in C.glutamicum und anderen coryneformen Bakterien zu Letalität auf Medien mit 10 % Sucrose führt.

Kolonien mit einem inaktivierten sacB-Gen können daher auf derartigen Medien positiv selektioniert werden, da in diesen Fällen die Inaktivierung durch Insertionselemente, die in sacB inseriert sind, bewirkt wird. Dieses wird dann durch eine Restriktionsanlayse lokalisiert.

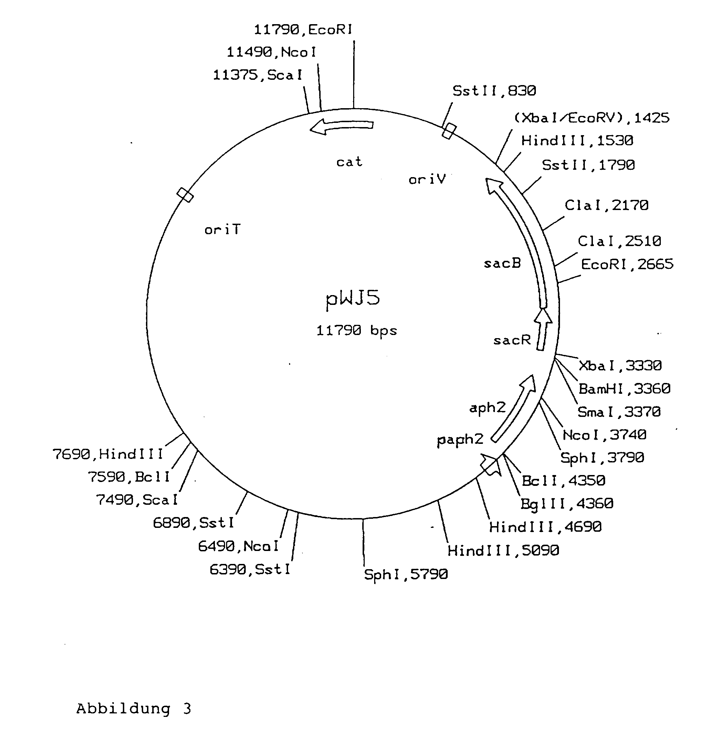

Bevorzugt eingesetzt wird der das sacB-Gen enthaltende Fangvektor pWJ5, dessen Restriktionskarte in Abb. 3 wiedergeben wird.

Er leitet sich aus den Plasmiden pECM1 (DE-OS 3841453) und pUM24 (Ried und Collmer, Gene 57, (1987) 239-246) ab.

Nach dem Restringieren des Plasmids pUM24 mit den Enzymen BamHI und EcoRV entsteht ein ∼ 1,9 kb großes DNA-Fragment, welches das sacB-Gen trägt und bevorzugt eingesetzt wird.

Auf diesem Weg wurde drei verschiedene, für die jeweiligen Bakteriengattungen offensichtlich charakteristische IS-Elemente in einer Reihe von coryneformen Bakterien gefunden, die nach ihrer Herkunft als ISCg1, ISBl1 und ISRf1 bezeichnet werden (Tabellen 1 und 2). Dabei kann der Wirtsbereich über die Gattung des Bakteriums, in dem das jeweilige IS-Element gefunden wurde, hinausgehen.

Durch Hybridisierung Digoxygenin-d-UTP markierter DNA von IS-Elementen, die nach dem erfindungsgemäßen Verfahren identifiziert wurden, gegen z. B. mit EcoRI oder einer anderen geeigneten Endonuclease gespaltene Gesamt-DNA aus dem zu untersuchenden Mikroorganismus werden dann eventuell vorhandene weitere Kopie dieser IS-Elemente im Genom dieses Stammes nachgewiesen.

Gegenstand der Erfindung sind somit insbesondere die in Tabelle 2 aufgeführten IS-Elemente, für die besonders charakteristisch die Gesamtlänge, die Länge IR und die Länge DR sind.

In den Sequenzen der invers repetitiven Enden können natürlich Basen äquivalent ersetzt sein, ohne daß sich die Wirksamkeit ändert, in der Weise, wie es dem Fachmann auch geläufig ist. Das gilt ebenso für die identifizierte Nucleotidsequenz von ISCg1 (Tab. 2/1, 2/2).

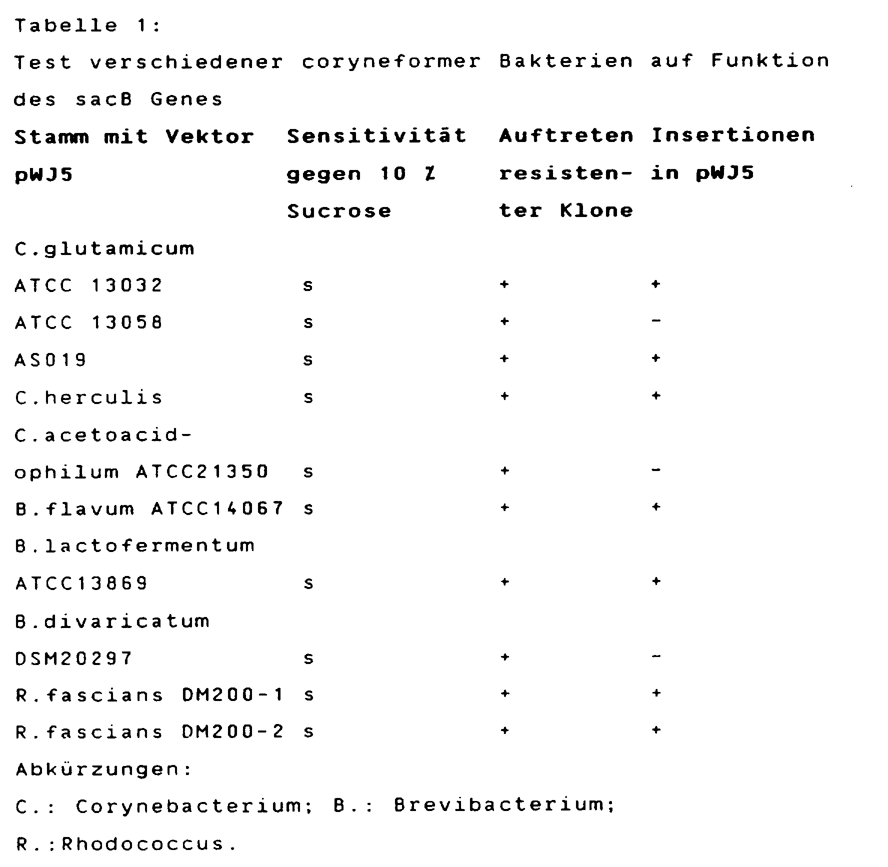

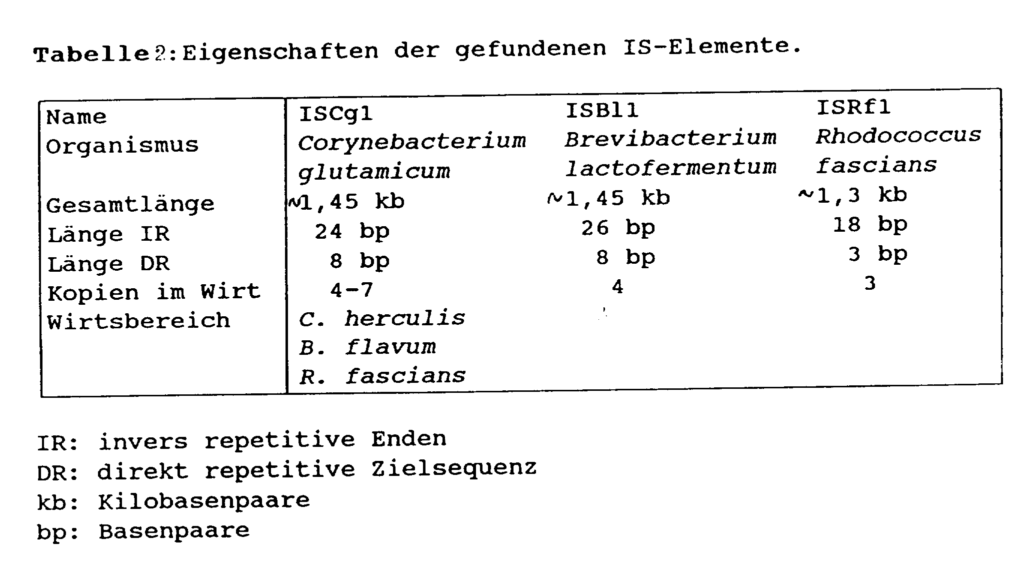

Sequenzen der invers repetitiven Enden*)**)

IR-L G G C c C T T C C g G T T T T g G g G T a C A T c a
ISCg1
IR-R G G C t C T T C C t G T T T T a G a G T g C A T t g

IR-L G G C T C T T C C G T T t T T A G A G T G C A T T G
ISBl1
IR-R G G C T C T T C C G T T g T T A G A G T G C A T T G

IR-1 G G a C C t G A C C C C c A T t T G
ISRf1
IR-2 G G g C C c G A C C C C g A T a T G

*): Kleinbuchstaben symbolisieren nichthomologe Basenpaare.
**): ISCg1 und ISBl1 besitzen ca. 75% Sequenzhomologie.

Anhand einer Mutante von Corynebacterium glutamicum ATCC13032 kann die Wirkung eines IS-Element demonstriert werden.

Die C.glutamicum Mutante LT 5.5 ist eine direkt vom C.glutamicum Wildtyp-Stamm ATCC13032 abgeleitete, spontan S-(2-Aminoethyl)-cystein (AEC) resistente Mutante, die einen Defekt in der Lysinaufnahme aufweist. Das lysI-Gen ist das in C.glutamicum für die Lysinaufnahme verantwortliche Gen. Dieses und die angrenzenden DNA Regionen sind kloniert und die Nukleotidsequenz des lysI-Gens bekannt.

Seep-Feldhaus, A.-H., Kalinowski, J. und Pühler, A. (1991) Mol. Microbiol. 5:2995-3005.

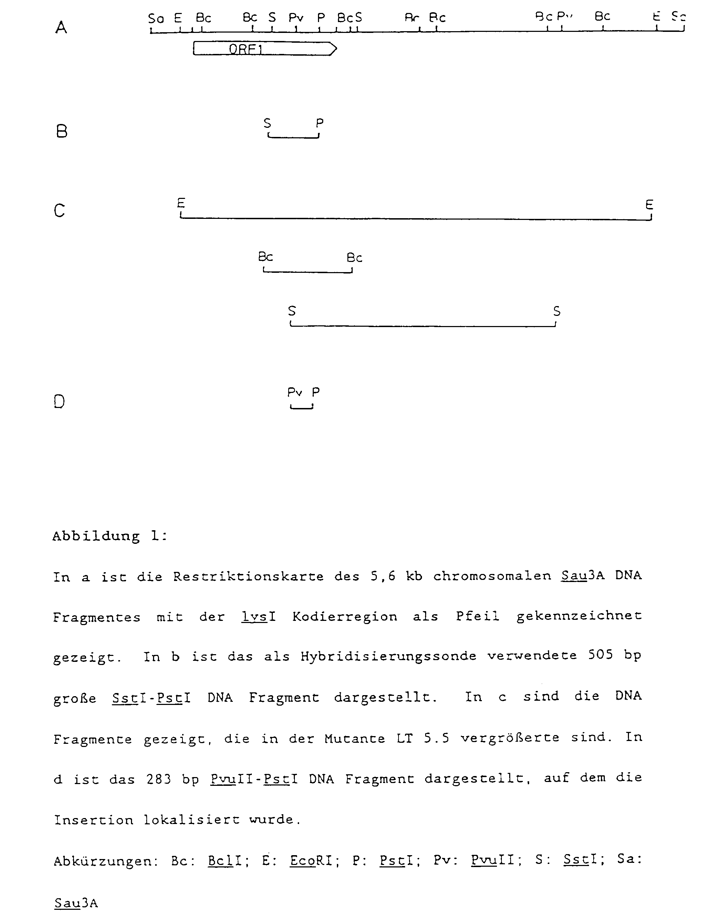

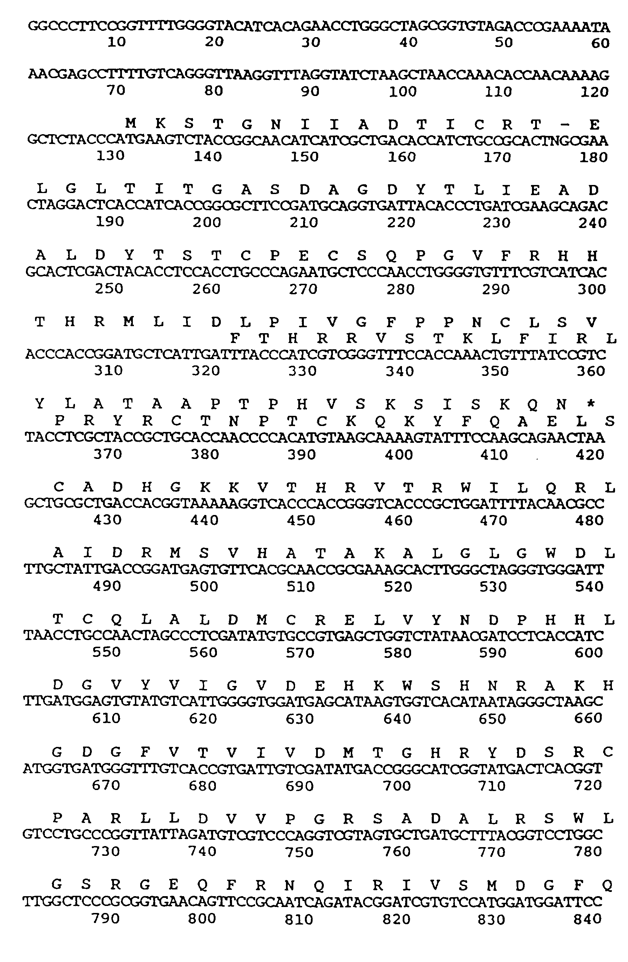

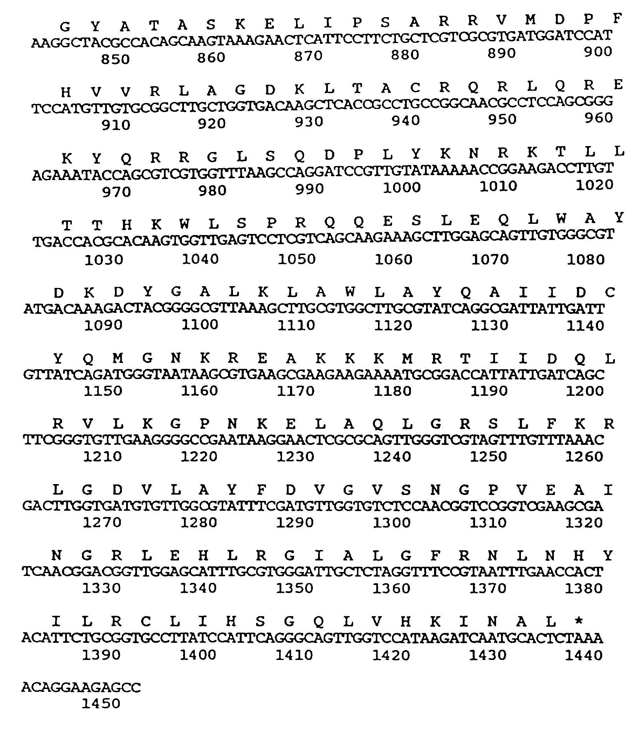

Hybridisiert man ein mit Dioxygenin-d-UTP-markiertes, 505 bp SstI-PstI DNA Fragment aus der lysI Kodierregion gegen Gesamt-DNA aus dem C.glutamicum Wildtyp-Stamm ATCC13032 und der Mutante LT 5,5, findet man im Wildtyp ein 5 kb großes EcoRI DNA-Fragment, während in der Mutanten LT 5.5 ein ca. 6,5 kb großes EcoRI DNA-Fragment hybridisiert. Die Mutation in dem lysI-Gen der Mutante LT 5.5 ist nach diesem Ergebnis auf die Insertion eines ca. 1,45 kb großen DNA-Fragmentes ISCg1 zurückzuführen, dessen Basensequenz aufgeklärt wurde (Abb. 2/1, 2/2).

Mit Hilfe des erfindungsgemäßen Verfahrens gelingt die Bestätigung des Vorhandenseins dieses auf klassischem Wege gefundenen IS-Elements ISCg1. Gleichzeitig werden fünf IS-Elemente in C.glutamicum ATCC13032 gefunden, die mit Digoxygenin-d-UTP markierter IsCg1-DNA hybridisieren und daher als IS-Elemente vom Typ ISCg1 bezeichnet werden.

Unter Verwendung der erfindungsgemäß aufgefundenen IS-Elemente läßt sich eine Anzahl von bestehenden Problemen lösen:
Das bisher bei Bakterien angewandte Verfahren mittels Chemikalien Mutagenesen zu erzeugen, hat den Nachteil, daß oft neben der gewünschten noch mehrere andere Mutationen in einer Zelle gesetzt werden, die sich u.U. ungünstig auswirken. Zudem sind die mutierten Gene nicht physikalisch markiert, da chemische Agentien unter den verwendeten Bedingungen meist nur Punktmutationen (Basenaustausch) bewirken.

Solch ein Basenaustausch oder auch mehrere sind in der Regel nur dazu geeignet, einzelne Gene nicht aber ganze Transkriptionseinheiten abzuschalten.

Transposonen und Insertionselemente schaffen hierbei insofern Abhilfe, daß pro Zelle im allgemeinen nur ein einzelnes Mutationsereignis erzeugt wird, und eine so erzeugte Mutation physikalisch markiert ist. Diese Markierung besteht entweder in einem selektionierbaren Marker auf dem Transposon oder neben einem Insertionselement oder, bei nicht solcherart konstruierten Insertionselemente, zumindest in einer deutlichen Verlängerung des mutagenisierten Bereichs durch eine bekannte Sequenz, die mittels DNA-Hybridisierung identifiziert werden kann.

Die mutagene Aktivität von IS-Elementen kann genutzt werden, indem ein selektionierbares Gen (z. B. ein Antibiotika-Resistenzgen) durch Klonierung nach bekannten Verfahren in oder zwischen zwei Kopien eines IS-Elements plaziert wird. Das so entstandene (composite) Transposon kann zur Mutagenese benutzt werden, wobei das mutierte Gen durch das selektionierbare Gen physikalisch markiert ist.

Damit wird der Einsatzubereich der Transposonmutagenese erweitert.

Bei der detaillierten Analyse von bekannten C.glutamicum Transposon-Mutanten stellt sich heraus, daß in manchen Fällen auch endogene IS-Elemente ihren Platz im Genom von C.glutamicum gewechselt haben. Es ist dann nicht eindeutig festzustellen, ob das Transposon oder ein Insertionselement die phänotypisch zu beobachtende Mutation erzeugt hat. Endogene Insertionselemente können also einen störenden Hintergrund bei der Transposon-Mutagenese darstellen.

Das erfindungsgemäße Verfahren gestattet es nun, IS-Elemente schnell zu identifizieren und durch DNA-Hybridisierung gegen die Gesamt-DNA in einem Transposon-Mutantenstamm festzustellen, ob das Insertionselement-Muster verändert ist, und damit die Gefahr besteht, daß die beobachtete Mutation durch ein Insertionselement ausgelöst wurde.

Ebenso gewährleistet die vollständige Entfernung von endogenen Insertionselementen durch "gene-replacement"-Techniken einen Insertionselementfreien Stamm, in dem phänotypische Mutationen eindeutig dem insertierten Transposon zugeordnet werden können. Mit der Verwendung der identifizierten Insertionselemente als Hybridisierungssonden bzw. der sacB-Technik zur Identifizierung von IS-Elementen können IS-Element-freie Stämme gefunden werden, die sich besser zur Transposon-Mutagenese eignen.

Unter optimalen Wachstumsbedingungen liegt die Transpositionsrate von IS-Elementen gewöhnlich unter 1×10⁻⁷ pro Generation. Sie wird allerdings deutlich erhöht, wenn der Mikroorganismus durch Änderungen des äußeren Milieus oder durch Destabilisierung des inneren metabolischen Gleichgewichts unter Streß gesetzt wird. Äußere Streßfaktoren stellen z. B. Hitze, Kälte, Nährstoffmangel oder antibiotische Substanzen wie Aminosäure-Analoga dar (Craig & Kleckner 1987).

Gerade rekombinante Mikroorganismen oder auxotrophe bzw. auf Hochproduktion selektierte Mutanten weisen ein destabilisiertes inneres Milieu auf und sind aus diesem Grund einer erhöhten Transpositionsfrequenz ausgesetzt.

Dieser Mechanismus kann auch positiv genutzt werden:

Durch Einsatz der nach dem erfindungsgemäßen Verfahren gefundenen Insertionselemente können beispielsweise angrenzende DNA-Regionen vervielfältigt werden.

Die IS-Elemente können auch mittels Replikonfusion zur Mutagenese eingesetzt werden. Dabei wird die Replikonfusion und damit die erzeugte Mutation durch Selektion auf diese Fusion stabilisiert. Im Falle einer Fusion zwischen dem bakteriellen Chromosom und einem IS-Element tragenden aber nicht replikationsfähigen Resistenzplasmid dient die Resistenz des Plasmids zur Selektion auf die stabile Replikonfusion.

Durch die Entfernung endogener IS-Elemente nach dem erfindungsgemäßen Verfahren können die oben angesprochenen Instabilitäten bei Produktionsstämmen bzw. unter Produktionsbedingungen wesentlich reduziert werden.

Mutagenese und Entfernung endogener IS-Elemente setzen im Prinzip bei dem aus dem DE-PS 4027453 bekannten Verfahren an, bei dem man einen mobilisierbaren E.coli-Vektor, wie er z. B. in Anspruch 1 dieser Erfindung unter den Kennzeichen 1.1.1 bis 1.1.3 aufgeführt wird, der aber als Kennzeichen 1.1.4 ein das entsprechende IS-Element enthaltendes DNA-Fragment aufweist, durch konjugativen Transfer aus einem E.coli Mobilisatorstamm in das gewünschte coryneforme Bakterium transferiert.

### Beispiel 1

### Mutationsauslösung durch IS-Elemente in Corynebacterium glutamicum: Nachweis und Isolierung eines IS-Elementes aus dem lysI-Gen der Mutante LT 5.5

Die C.glutamicum Mutante LT 5.5 ist eine direkt vom c.glutamicum Wildtyp-Stamm ATCC 13032 abgeleitete, spontan S-(2-Aminoethyl)-cystein (AEC) resistente Mutante, die einen Defekt in der Lysinaufnahme aufweist. Das lysI-Gen ist das in C.glutamicum für die Lysinaufnahme verantwortliche Gen. Das lysI-Gen und die angrenzenden DNA Regionen sind kloniert und die Nukleotidsequenz des lysI-Gens bekannt (Seep-Feldhaus, A. H. et al., Mol. Microbiol. 5(12): 2995-3005, 1991).

Ein mit Dioxygenin-d-UTP-markiertes, 505 bp SstI-PstI DNA Fragment aus der lysI Kodierregion (Abbildung 1) wurde gegen Gesamt-DNA aus dem C.glutamicum Wildtyp-Stamm ATCC 13032 und der Mutante LT 5.5 hybridisiert. Die Gesamt-DNA, isoliert nach der Methode von Altenbuchner und Cullum (Mol.Gen.Genet. 195: 134-138, 1984), wurde zu diesem Zweck mit dem Restriktionsenzym EcoRI gespalten. Die Spaltungsansätze wurden anschließend in einem 0,8% Agarosegel aufgetrennt. Der Transfer der DNA Fragmente auf eine Nylonmembran (Hybond-N, Amersham, Braunschweig) erfolgte nach der Methode von Southern (J.Mol.Biol. 98: 503-517, 1975). Die Hybridisierung wurde mit dem "DNA Labeling and Detection Kit nonradioactive" (Boehringer, Mannheim) durchgeführt.

Das als Hybridisierungssonde verwendete SstI-PstI DNA Fragment hybridisiert im C.glutamicum Wildtyp-Stamm ATCC 13032 mit einem 5 kb großes EcoRI DNA Fragment, während in der Mutante LT 5.5 ein ca. 6,5 kb großes EcoRI DNA Fragment hybridisiert. Die Mutation in dem lysI-Gen der Mutante LT 5.5 ist nach diesem Ergebnis auf die Insertion eines ca. 1,5 kb großen DNA Fragmentes zurückzuführen.

Zur Bestimmung des Insertionsortes des in das lysI Gen der Mutante LT 5.5 inserierten DNA Fragmentes wurde die Gesamt-DNA des C.glutamicum Wildtyp-Stammes ATCC 13032 und der Mutante LT 5.5 parallel mit den Restriktionsenzymen PvuII und SstI gespalten. Die Spaltungsansätze wurden anschließend im 0,8% Agarosegel aufgetrennt. Der Transfer der DNA Fragmente und die Hybridisierung mit dem Dioxygenin-d-UTP-markierten SstI-PstI DNA Fragment aus der lysI Kodierregion erfolgte wie oben beschrieben. Die Hybridierung zeigt, daß in der Mutante LT 5.5 sowohl das 2,8 kb PvuII DNA Fragment als auch das 0,9 kb SstI DNA Fragment (Abbildung 1) um ca. 1,5 kb verlängert ist. Aus den Hybridisierungen folgt, daß die Insertion auf dem 283 bp PvuII-PstI DNA Fragment der lysI Kodierregion lokalisiert ist (Abbildung 1).

Zur Klonierung der Insertion wurde das, in der Mutante LT 5.5 vergrößerte PvuII-PstI DNA Fragment zunächst mit Hilfe der "Polymerase Chain Reaction" (Innis, M., A. et al., PCR Protokoll, Academic Press, 1990) amplifiziert. Die für die PCR-Reaktion eingesetzten Primer sind 20 Basen lange Oligonukleotide mit der folgenden, aus der lysI DNA Sequenz abgeleiteten Sequenz:

Primer 1: 5' CAAAATCGGGGCCATCAACA 3'
Primer 2: 5' GAGGACAAACTGCGGTTCTG 3'

Die PCR-Reaktion wurde mit folgendem Ansatz durchgeführt:
- 500 ng Gesamt-DNA aus der Mutante LT 5.5
- 14 ng Primer 1
- 14 ng Primer 2
- 200 µM d-NTP (dATP, dCTP, dGTP, dTTP)
gelöst in einem Volumen von 50 µl Taq-Polymerase Reaktionspuffer (Boehringer, Mannheim). Die PCR Reaktion wurde mit dem Gene ATAQ Controller (Pharmacia) durchgeführt. Der Ansatz wurde zunächst 5 min bei 96 °C inkubiert. Nach Zugabe von 2,5 unit Taq-Polymerase (Boehringer, Mannheim) wurde dann für die Amplifikation des 1,8 kb PvuII-PstI DNA Fragmentes folgender Zyklus 30 mal durchlaufen:
- 1 min 10 sec 53°C
- 2 min 40 sec 72°C
- 1 min 10 sec 92°C

Die amplifizierte DNA wurde in einem 0,8% Agarosegel aufgetrennt und aus dem Gel isoliert (Geneclean BIO101 Inc., La Jolla, California). Diese DNA wurde mit den Restriktionsenzymen PstI und PvuII gespalten und mit dem PstI und SmaI gespaltenen E.coli Plasmidvektor pK18mob (Patentanmeldung P4027453.5) ligiert. Mit dem Ligationsgemisch wurde der E.coli Stamm DH5α (Woodcock, D., M. et al., Nucleic Acids Res. 17: 3469-3478, 1989) transformiert. Aus den Transformaten konnte ein Plasmid, genannt pSF3, isoliert werden, welches aus dem Vektor pK18mob und aus dem 1,8 kb langen amplifiziertem DNA Fragment besteht.

### Beispiel 2

### Sequenzanalyse des IS-Elementes aus C.glutamicum

Das im Plasmid pSF3 (Beispiel 1) klonierte, etwa 1.8 kb große DNA-Fragment aus der *C.glutamicum*-Mutante LT 5.5 wurde nach der Methode von Sanger *et. al.* Proc. Natl. Acad. Sci. USA 74:5463-5467, 1977) mit den Modifikationen für die Sequenzierung doppelsträngiger DNA (Chen und Seeburg, DNA 4:165-168, 1985) sequenziert.

Dazu wurden mittels durch Restriktionskartierung bestimmter Schnittstellen Deletionsderivate des Plasmids pSF3 hergestellt . Da das Plasmid pK18mob zur direkten Sequenzierung geeignet ist, konnten diese Deletionsderivate umgehend zur Sequenzierung eingesetzt werden.

Die Sequenzierung erfolgte mit dem T7-sequencing kit (Pharmacia, Freiburg) und den Primern "universal" bzw. "reverse". Die Nukleotidsequenz wurde von beiden DNA-Strängen vollständig bestimmt (Abbildung 2).

Das IS-Element ISCg1 ist 1452 Basenpaare (bp) lang und hat imperfekte invers repetitive Enden von 24 bp Länge. An der Insertionsstelle im lysI-Gen erzeugt es eine direkt repetitive Sequenz von 8 bp. Es trägt zwei offene Leseraster (ORF), die wahrscheinlich für Proteine kodieren. ORF1 (bp 130 bis 417 kodiert für ein Protein von 96 Aminosäuren und ORF2 (bp 321 bis 1436) kodiert für ein Protein von 372 Aminosäuren. ORF1 beginnt mit einem ATG-Startkodon, vor dem sich eine Sequenz mit Ähnlichkeit zu Ribosomenbindungsstellen Gram-positiver Bakterien befindet (bp 117-121 5'-AAAGG-3'). ORF2, der das Ende von ORF1 überlappt, besitzt zwar einige interne mögliche ATG- oder GTG-Startkodons, vor diesen befinden sich allerdings keine sichtbaren Ribosomenbindungsstellen.

### Beispiel 3:

### Konstruktion eines Vektors für die Isolierung von Insertionssequenzen aus coryneformen Bakterien

Für die Isolierung von Insertionssequenzen (IS-Elementen) oder Transposons wird das *sac*B-Gen aus *Bacillus subtilis* verwendet (Gay *et al.*, J.Bacteriol. 153:1424-1431, 1983). Das Gen kodiert für das Exoenzym Levansucrase, welches die Reaktionen Saccharose-Hydrolyse und Levan-Synthese katalysiert (Dedonder *et al.,* Methods in Enzymol. 8:500-505, 1966). Die Expression von *sac*B in *E.coli* führt zum Transport des Enzyms ins Periplasma (Steinmetz *et al.,* Mol.Gen.Genet. 191:138-144, 1983) und ist für *E.coli* und andere Gram-negative Bakterien letal auf Medien mit über 5% Sucrose (Gay *et al.*, J.Bacteriol. 164:918-921).

In dieser Erfindung wird das *sac*B-Gen zum Auffinden von Insertionssequenzen in coryneformen Bakterien eingesetzt. Die Expression des intakten *sac*B-Genes führt auch in C.glutamicum und anderen coryneformen Bakterien zu Letalität auf Medien mit 10% Sucrose (Beispiel 4). Kolonien mit einem inaktivierten *sacB*-Gen können daher auf derartigen Medien positiv selektioniert werden. Man erhält so die Möglichkeit, Insertionselemente, die in *sac*B inseriert sind aufzufinden. Der IS-Fangvektor pWJ5 (Abb. 3) ist ein Derivat des Plasmides pECM2. Zur Herstellung des Plasmides pECM2 wurde das Plasmid pECM1 (Schäfer *et al.,* J.Bacteriol. 172:1663-1666, 1990)¹ mit dem Restriktionsenzym *Sal*I gespalten und mit T4-DNA-Ligase religiert. Dabei wurde ein Derivat, dem das 0,3kb *Sal*I-Fragment von pECM1 fehlt erhalten und mit pECM2 bezeichnet. Der IS-Fangvektor wurde wie folgt hergestellt: Plasmid pUM24 (Ried und Collmer, Gene 57:239-246, 1987) wurde mit den Restriktionsenzymen *Bam*HI und *Eco*RV restringiert, wodurch ein 1,9kb großes DNA-Fragment entsteht, welches das *sac*B-Gen trägt. Das Plasmid pECM2 wurde mit dem Restriktionsenzym *Xba*I gespalten und anschließend mit dem Enzym Klenow-Polymerase behandelt, um die an der Spaltungsstelle entstandenen überstehenden Einzelstrang-Enden abzudauen (Maniatis *et al*., Molecular cloning 1,2nd ed., 5.42, 1989). Die so behandelte DNA wurde durch Phenolisierung und Alkoholfällung gereinigt und konzentriert, und anschließend mit dem Restriktionsenzym *Bam*HI gespalten. Beide Ansätze wurden vereinigt und mit T4-DNA-Ligase ligiert. Mit dem Ligationsgemisch wurden kompetente Zellen des *E.coli*-Stammes S17-1 (Simon *et al*., Biotechnol. 1:784-794, 1983) transformiert. Die Selektion transformierter Klone erfolgte primär auf PA-Agar (17,5g Penassay Broth + 15g Agar auf 1l Medium) mit 50µg/ml Chloramphenicol. Resistente Kolonien wurden durch paralleles Auftragen auf LB-Medium mit 10% Sucrose auf eine erfolgreiche Klonierung des *sac*B-Genes, erkennbar am sensitiven Phänotyp, nachgetestet.
¹≙ EP-A - 0 372 230

### Beispiel 4:

### Isolierung von IS-Elementen aus coryneformen Bakterien

Der Nachweis und die Isolierung von Insertionselementen aus coryneformen Bakterien in großem Maßstab gelang durch Anwendung des *sac*B-Systems:

Der mobilisierbare Shuttle-Vektor pWJ5 (Beispiel 3) wurde durch Konjugation (DE-OS 3841453.8; Schäfer *et al*., J.Bacteriol. 172: 1663-1666 (1990)) aus dem Mobilisatorstamm *E.coli* S17-1 (Simon *et al*., Biotechnology 1: 784-794 (1983)) in insgesamt 20 coryneforme Rezipientenstämme aus den Gattungen *Arthrobacter*, *Brevibacterium*, *Corynebacterium*, *Microbacterium* und *Rhodococcus* übertragen (Tabelle 1). Die Anwesenheit unveränderter pWJ5-Plasmide in den coryneformen Stämmen wurde durch Lyse der Transkonjuganten (Birnboim & Doly Nucl. Acids Res. 7: 1513-1523 (1979)), Spaltung der Plasmide mit den Restriktionsendonukleasen *Bam*HI und *Ssp*I und Analyse der Fragmente im Agarosegel verifiziert. Jeder der 20 getesteten Stämme zeigte in anschließenden Tests Wachstum auf LB-Medium mit Km₂₅, aber kein Wachstum auf LBKm₂₅-Medium mit 10% Sucrose (Tabelle 1). Diese Sucrose-Sensitivität ist auf die Expression des intakten *sac*B-Genes auf dem Plasmid pWJ5 zu zurückzuführen, da Stämme mit dem Plasmid pECM2 (Beispiel 3) auf LBkm₂₅ mit 10% Sucrose wachsen können. pWJ5 tragende Einzelkolonien der zu testenden Stämme wurden in LB-Flüssigmedium bis zum Erreichen der logarithmischen Wachstumsphase bei 30°C im Luftschüttler inkubiert. Jeweils etwa 5x10⁹ Zellen wurden anschließend durch Zentrifugation für 10min bei 3000 U/min geerntet, das Pellet in 1ml LB-Medium aufgenommen und auf die Oberfläche eines auf LB-Medium plazierten 0,45µm Celluloseacetatfilter (Durchmesser 40mm, Sartorius, Göttingen, FRG) aufgebracht. Nach dem Eintrocknen erfolgte eine Inkubation der Zellen für 20h bei 38,5°C. Die Filter wurden danach mit 1ml LB-Medium abgeschwemmt und je 0,1ml der unverdünnten und der im Verhältnis 1:10 mit LB-Medium verdünnten Suspension auf LBKm₂₅-Agar
mit 10% Sucrose ausplattiert. Nach 2-3 tägiger Aufbewahrung bei 30°C konnten für alle coryneformen Bakterienstämme sucroseresistente Kolonien in unterschiedlicher Häufigkeit erhalten werden (Tabelle 1). Für *Corynebacterium glutamicu*m wurden pro Ansatz 2,5x10⁵ Sucrose-resistente Kolonien pro Ansatz, entsprechend einer Frequenz von 5x10⁻⁵ erhalten. 16 Sucrose-resistente Klone wurden exemplarisch durch Lyse, Restriktion der Plasmide mit den Enzymen BamHI und *Ssp*I und anschließender Agarose-Gelelektrophorese untersucht. In 8 Klonen konnte eine etwa 1,45kb große Verlängerung des *sac*B-Genes im Plasmid pWJ5 nachgewiesen werden. Sämtliche Insertionen wiesen Erkennungsstellen für die Restriktionsendonukleasen *BamHI*, *BclI*, *NcoI*, *Hind*III und *DraI* auf, die auch im Insertionselement ISCg1 (Beispiel 2) vorhanden sind. Alle getesteten Insertionen hybridisierten mit Digoxygenin-dUTP markierter ISCg1-DNA. Mit Hilfe des IS-Fangvektors konnten Insertionen vom ISCg1-Typ zudem aus *C.glutamicum* AS019, *C.fascians* DM200-2, *C.fascians* DM 200-1, *C.herculis* und *B.flavum* ATCC 14067 isoliert werden (Tabelle 1).

Es wurde überdies eine Hybridisierung *Eco*RI-gespaltener chromosomaler DNA verschiedener coryneformer Stämme mit dem Digoxigenin-dUTP markierten 1,9kb Fragment aus der PCR-Reaktion (Beispiel 2) durchgeführt. Die Hybridisierung zeigt, daß in *C.glutamicum* ATCC 13032 und in der Mutante LT5.5 jeweils 5 Kopien von ISCg1 oder eines sehr ähnlichen Elementes auftreten. Weiterhin wurde ISCg1 oder ein sehr ähnliches Element mittels Hybridisierung in den Stämmen *C.glutamicum* AS019 und *Brevibacterium flavum* DSM 20411 identifiziert. Durch Hybridisierung konnten keine Kopien von ISCg1 in *C.glutamicum* ATCC 13058, *C.acetoacidophilum* ATCC 21350, *Brevibacterium divaricatum* DSM 20297 und in *E.coli* K12 nachgewiesen werden. Die durch Hybridisierung ermittelten Resultate sind somit in Übereinstimmung mit der mit Hilfe des IS-Fangvektors pWJ5 ermittelten Verteilung von IS-Elementen vom ISCg1-Typ in coryneformen Bakterienstämmen.

Eine Insertion anderen Typus wurde aus *B.lactofermentum* ATCC 13869 isoliert (Tabelle 1) und ISBl1 genannt. Diese etwa 1,4kb große Insertion befand sich ebenfalls im *sac*B-Gen von pWJ5 und weist lediglich partielle Homologie zu ISCg1 auf. Durch Hybridisierung Digoxygenin-dUTP markierter ISBl1-DNA gegen *Eco*RI gespaltene Gesamt-DNA aus *B.lactofermentum* ATCC 13869 wurden 4 Kopien von ISBl1 im Genom dieses Stammes nachgewiesen.

Eine etwa 1,3kb große Insertionssequenz wurde überdies in *Rhodococcus fascians* DM200-2 (früher *Corynebacterium fascians*) identifiziert (Tabelle 1) und mit ISRf1 bezeichnet. Restriktionsanalysen und Hybridisierungsstudien zeigten nur geringe Ähnlichkeiten zur ISCg1-Familie und zu ISBl1. Durch Hybridisierung Digoxygenin-dUTP markierter ISRf1-DNA gegen *Pst*I-gespaltene Gesamt-DNA aus *R.fascians* DM200-2 wurden 3 Kopien von ISRfI im Genom des Stammes nachgewiesen. Eine Kopie von ISRf1 konnte auf einem endogenen Plasmid von *R.fascians* DM200-2 lokalisiert werden.

Wesentliche Eigenschaften der drei isolierten IS-Element-Typen aus coryneformen Bakterien sind in Tabelle 2 vergleichend zusammengestellt.

### Beispiel 5:

### Mutagenese von coryneformen Stämmen durch Einsatz von Insertionselementen

Das Prinzip der Mutagenese mit Insertionselementen wird am Beispiel des Stammes *C.glutamicum* ATCC 13058 und dem IS-Element ISBl1 (Beispiel 4) dargelegt:

Durch Restriktionsanalyse wurde eine Insertion von ISBl1 in einem 0,65kb großen *Hind*III-*ClaI*-Fragment des *sac*B-Genes auf pWJ5 lokalisiert. Durch Restriktion des pWJ5::ISBl1-Plasmides mit den Restriktionsendonukleasen *Hin* dIII und *Cla*I wurde ein 2,05kb großes Fragment freigesetzt und nach Auftrennung durch Agarose-Gelelektrophorese aus dem Gel isoliert. Hierzu wurde ein schmaler Streifen mit dem entsprechenden Fragment. ausgeschnitten und mit 2,5-3 Volumen 6 molarer Natriumjodid-Lösung versetzt. Nach 10 minütiger Inkubation bei 55°C wurde die DNA aus dem nun geschmolzenen Gel mit Hilfe des GeneClean Kits (BIO101 Inc. La Jolla, CA. USA) nach Angaben des Herstellers isoliert und gereinigt. Anschließend wurden die überstehenden Einzelstrang-Enden des Fragmentes mit dem Enzym Klenow-Polymerase aufgefüllt.

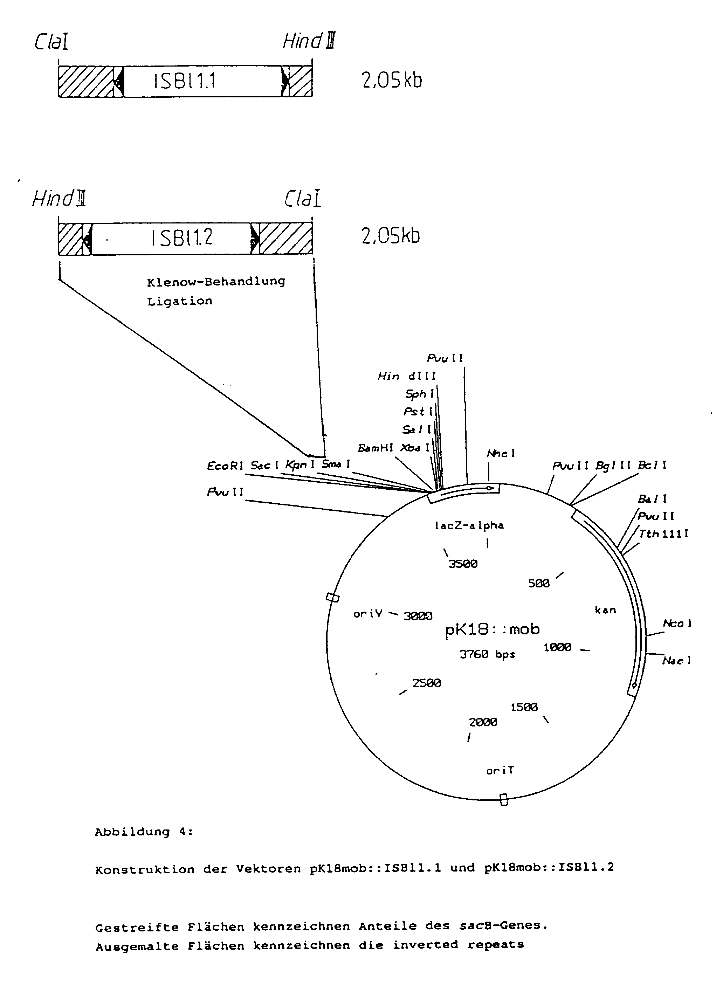

Der mobilisierbare *E.coli*-Vektor pK18mob (DE-OS 4027453) wurde mit dem Restriktionsenzym *Sma*I linearisiert und nach dem Fachmann geläufigen Verfahren (Maniatis *et al*., Molecular cloning 2nd ed. Abschnitt 1.6, Gold spring Harbor Laboratory Press, 1989) mit dem Enzym alkalische Phosphatase behandelt. Der so behandelte Vektor wurde mit dem aufgefüllten *Hin* dIII-*Cla*I-Fragment vermischt und mit dem Enzym T4-DNA Ligase ligiert. Der Ligationsansatz wurde anschließend in den *E.coli*-Stamm Dh5α (Woodcock *et al*., Nucleic Acids Res. 17:3469-3478, 1989) transformiert. Transformanten wurden auf LB-Medium mit Kanamycin (50µg/ml) und 5-Brom-4-Chlor-3-Indolyl-β-D-Galctosid (20µg/ml) ausplattiert und für 24h bei 37°C inkubiert. Weiße Kolonien wurden auf LB-Medium mit Kanamycin (50µg/ml) gereinigt und der Plasmidgehalt durch Lyse der Zellen, Restriktion der Plasmid-DNA und Agarosegelelektrophorese analysiert. Klone, die das 2,05kb große Insert mit ISBl1 in beiden Orientierungen tragen wurden identifiziert und mit pK18mob::ISBl1.1 und pK18mob::ISBl1.2 bezeichnet (Abbildung 4). pK18mob::ISBl1.1 wurde aus dem Stamm *E.coli* Dh5α isoliert und in kompetente Zellen des Stammes *E.coli* S17-1 transformiert.

Aus dem Mobilisatorstamm *E.coli* S17-1 wurde das Plasmid pK18mob::ISBl1.1 durch konjugativen Transfer (Schäfer *et al*., J.Bacteriol. 172:1663-1666, 1990, EP-A-0372230) bei auf 38,5°C erhöhter Inkubationstemperatur nach *C.glutamicum* ATCC 13058 übertragen. Auf Selektionsmedium (LBKm₂₅Nx₅₀) wurden 1500 Transkonjuganten erhalten. 12 dieser Klone wurden exemplarisch auf ihren Plasmidgehalt hin überprüft. Dabei wurde keine freie Plasmid-DNA nachgewiesen. 400 Transkonjuganten wurden parallel auf LBKm₂₅Nx₅₀- und MMKm₂₅Nx₅₀-Medium gestochert und für 2-3 Tage bei 30°C inkubiert. 3 Klone erwiesen sich als auxotroph, da kein Wachstum auf Minimalmedium zu beobachten war. 12 zufällig ausgewählte Transkonjugantenklone wurden in LBKm₅₀-Medium angezogen und die Gesamt-DNA isoliert, mit dem Enzym *Eco*RI gespalten und im Agarosegel aufgetrennt. Durch Hybridiserung mit Digoxigenin markierter *Eco*RI-gespaltener pK18mob::ISBl1.1-DNA konnte eine Integration des Vektors an unterschiedlichen Stellen in das Genom von *C.glutamicum* ATCC 13058 verifiziert werden.

## Patentansprüche

1. Verfahren zum Auffinden von Insertionselementen (IS-Elemente) oder Transposonen in coryneformen Bakterien, das umfaßt:
1.1. die Konstruktion eines aus einem E.coli Mobilisatorstamm mobilisierbaren, nicht selbstransferierbaren Vektors, zusammengesetzt aus
1.1.1 einem DNA-Segment, enthaltend ein in E.coli funktionelles Replikon,
1.1.2 einem zweiten DNA-Segment, enthaltend das für die Mobilisierungfunktion codierende DNA-Fragment (Mob-site enthaltend den oriT),
1.1.3 einem dritten DNA-Segment, das in Gram-positiven Bakterien homolog rekombiniert und/oder ein in coryneformen Bakterien funktionelles Replikon enthält
1.1.4 einem das sacB-Gen enthaltenden DNA-Segment aus Bacillus subtilis,
1.2 Übertragung dieses Vektors durch konjugativen Transfer in die coryneformen Rezipientenstämme,
1.3. Anzucht der den Vektor enthaltenden Transkonjuganten in einem 10 % Sucrose enthaltenden Nährmedium
1.4 Lyse der Sucrose-resistenten Klone, Spaltung der Plasmide mit Restriktonsendonucleasen und Analyse der Fragmente.

2. Mobilisierbarer nicht selbsttransferierbarer Vektor zum Auffinden von Insertionselementen oder Transposonen in coryneformen Bakterien,der zusammengesetzt ist aus
a) einem DNA-Segment, enthaltend ein in E.coli funktionelles Replikon
b) einem zweiten DNA-Segment, enthaltend das für die Mobilisierungsfunktion codierende DNA-Fragment (Mob-site enthaltend den oriT),
c) einem dritten DNA-Segment, das in Gram-positiven Bakterien homolog rekombiniert und/oder ein in coryneformen Bakterien funktionelles Replikon enthält,
d) einem das sacB-Gen enthaltenden DNA-Segment aus Bacillus subtilis.

3. Vektor gemäß Anspruch 2,
dadurch gekennzeichnet, daß man den Vektor pWJ 5 (Fangvektor) einsetzt, der durch die in Abb. 3 wiedergegebene Restriktionskarte beschrieben wird und aus 11790 bp besteht

4. Insertionselement des Typs ISCg1 aus C. glutamicum, C.fascians, C.herculis und B.flavum, bestehend aus ca. 1,45 kb und mit den invers repetitiven Enden von ca. 24 bp Länge:

IR-L G G C c C T T C C g G T T T g G g G T a C A T c a

IR-R G G C t C T T C C t G T T T a G a G T g C A T t g

5. Insertionselement gemäß Anspruch 4,
dadurch gekennzeichnet, daß es aus 1452 bp besteht und die Basensequenz besitzt:

GGCCCTTCCGGTTTTGGGTACATCACAGAACCTGGCTAGCGGTGTAGACCCGAAAATA
10 20 30 40 50 60

AACGAGCCTTTTGTCAGGGTTAAGGTTTAGGTATCTAAGCTAACCAAACACCAACAAAAG
70 80 90 100 110 120

M K S T G N I I A D T I C R T - E
GCTCTACCCATGAAGTCTACCGGCAACATCATCGCTGACACCATCTGCCGCACTNGCGAA
130 140 150 160 170 180

L G L T I T G A S D A G D Y T L I E A D
CTAGGACTCACCATCACCGGCGCTTCCGATGCAGGTGATTACACCCTGATCGAAGCAGAC
190 200 210 220 230 240

A L D Y T S T C P E C S Q P G V F R H H
GCACTCGACTACACCTCCACCTGCCCAGAATGCTCCCAACCTGGGGTGTTTCGTCATCAC
250 260 270 280 290 300

T H R M L I D L P I V G F P P N C L S V
F T H R R V S T K L F I R L
ACCCACCGGATGCTCATTGATTTACCCATCGTCGGGTTTCCACCAAACTGTTTATCCGTC
310 320 330 340 350 360

Y L A T A A P T P H V S K S I S K Q N *
P R Y R C T N P T C K Q K Y F Q A E L S
TACCTCGCTACCGCTGCACCAACCCCACATGTAAGCAAAAGTATTTCCAAGCAGAACTAA
370 380 390 400 410 420

C A D H G K K V T H R V T R W I L Q R L
GCTGCGCTGACCACGGTAAAAAGGTCACCCACCGGGTCACCCGCTGGATTTTACAACGCC
430 440 450 460 470 480

A I D R M S V H A T A K A L G L G W D L
TTGCTATTGACCGGATGAGTGTTCACGCAACCGCGAAAGCACTTGGGCTAGGGTGGGATT
490 500 510 520 530 540

T C Q L A L D M C R E L V Y N D P H H L
TAACCTGCCAACTAGCCCTCGATATGTGCCGTGAGCTGGTCTATAACGATCCTCACCATC
550 560 570 580 590 600

D G V Y V I G V D E H K W S H N R A K H
TTGATGGAGTGTATGTCATTGGGGTGGATGAGCATAAGTGGTCACATAATAGGGCTAAGC
610 620 630 640 650 660

G D G F V T V I V D M T G H R Y D S R C
ATGGTGATGGGTTTGTCACCGTGATTGTCGATATGACCGGGCATCGGTATGACTCACGGT
670 680 690 700 710 720

P A R L L D V V P G R S A D A L R S W L
GTCCTGCCCGGTTATTAGATGTCGTCCCAGGTCGTAGTGCTGATGCTTTACGGTCCTGGC
730 740 750 760 770 780

G S R G E Q F R N Q I R I V S M D G F Q
TTGGCTCCCGCGGTGAACAGTTCCGCAATCAGATACGGATCGTGTCCATGGATGGATTCC
790 800 810 820 830 840

G Y A T A S K E L I P S A R R V M D P F
AAGGCTACGCCACAGCAAGTAAAGAACTCATTCCTTCTGCTCGTCGCGTGATGGATCCAT
850 860 870 880 890 900

H V V R L A G D K L T A C R Q R L Q R E
TCCATGTTGTGCGGCTTGCTGGTGACAAGCTCACCGCCTGCCGGCAACGCCTCCAGCGGG
910 920 930 940 950 960

K Y Q R R G L S Q D P L Y K N R K T L L
AGAAATACCAGCGTCGTGGTTTAAGCCAGGATCCGTTGTATAAAAACCGGAAGACCTTGT
970 980 990 1000 1010 1020

T T H K W L S P R Q Q E S L E Q L W A Y
TGACCACGCACAAGTGGTTGAGTCCTCGTCAGCAAGAAAGCTTGGAGCAGTTGTGGGCGT
1030 1040 1050 1060 1070 1080

D K D Y G A L K L A W L A Y Q A I I D C
ATGACAAAGACTACGGGGCGTTAAAGCTTGCGTGGCTTGCGTATCAGGCGATTATTGATT
1090 1100 1110 1120 1130 1140

Y Q M G N K R E A K K K M R T I I D Q L
GTTATCAGATGGGTAATAAGCGTGAAGCGAAGAAGAAAATGCGGACCATTATTGATCAGC
1150 1160 1170 1180 1190 1200

R V L K G P N K E L A Q L G R S L F K R
TTCGGGTGTTGAAGGGGCCGAATAAGGAACTCGCGCAGTTGGGTCGTAGTTTGTTTAAAC
1210 1220 1230 1240 1250 1260

L G D V L A Y F D V G V S N G P V E A I
GACTTGGTGATGTGTTGGCGTATTTCGATGTTGGTGTCTCCAACGGTCCGGTCGAAGCGA
1270 1280 1290 1300 1310 1320

N G R L E H L R G I A L G F R N L N H Y
TCAACGGACGGTTGGAGCATTTGCGTGGGATTGCTCTAGGTTTCCGTAATTTGAACCACT
1330 1340 1350 1360 1370 1380

I L R C L I H S G Q L V H K I N A L *
ACATTCTGCGGTGCCTTATCCATTCAGGGCAGTTGGTCCATAAGATCAATGCACTCTAAA
1390 1400 1410 1420 1430 1440

ACAGGAAGAGCC
1450

6. Isertionselemente des Typs ISBl1 aus B.lactofermentum bestehend aus ca. 1,45 kb und mit invers repetitiven Enden von ca. 26 bp Länge:

IR-L G G C T C T T C C G T T t T T A G A G T G C A T T G

IR-R G G C T C T T C C G T T g T T A G A G T G C A T T G

7. Insertitionselemente des Typs ISRF1 aus C.fascians, bestehend aus ca. 1,3 kb und mit invers repetitiven Enden von ca. 18 bp Länge:

IR-1 G G a C C t G A C C C C c A T t T G

IR-2 G G g C C c G A C C C C g A T a T G

8. Verwendung der Insertionselemente gemäß den Ansprüchen 5 bis 7 zur Mutagenese coryneformer Bakterien,
dadurch gekennzeichnet, daß man
8.1 einen aus einem E.coli Mobilisatorstamm mobilisierbaren, nicht selbsttransferierbaren Vektor konstruiert, zusammengesetzt aus
8.1.1. einem DNA-Segment, enthaltend ein in E.coli funktionelles Replikon
8.1.2 einem zweiten DNA-Segment, enthaltend das für die Mobilisierungsfunktion codierende DNA-Fragment (Mob-site enthaltend den oriT),
8.1.3 einem dritten DNA-Segment, das in Gram-positiven Bakterien homolog rekombiniert und/oder ein in coryneformen Bakterien funktionelles Replikon enthält, und
8.1.4. einem das IS-Element enthaltenden DNA-Segment
8.2 diesen Vektor durch konjugativen Transfer in den coryneformen Rezipientenstamm überträgt und
8.3. die den Vektor enthaltenden Transkonjuganten in dem gewünschten Nährmedium anzieht.

## Claims

1. Method for the detection of insertion elements (IS elements) or transposons in Coryneform bacteria, which comprises:
1.1 the construction of a vector which is mobilisable from an E. coli mobiliser strain and is not auto-transferable, composed of
1.1.1 a DNA segment, containing a replicon functional in E. coli,
1.1.2 a second DNA segment, containing the DNA fragment coding for the mobilisation function (mob site containing the oriT),
1.1.3 a third DNA segment, which recombines homologously in gram-positive bacteria and/or contains a replicon functional in Coryneform bacteria,
1.1.4 a DNA segment containing the sacB gene from Bacillus subtilis,
1.2 transfer of this vector by conjugative transfer into the Coryneform recipient strain,
1.3 cultivation of the transconjugant containing the vector in a nutrient medium containing 10% sucrose,
1.4 lysis of the sucrose-resistant clones, cleavage of the plasmids by restriction endonucleases and analysis of the fragments.

2. Mobilisable, not auto-transferable vector for the detection of insertion elements or transposons in Coryneform bacteria, which is composed of
a) a DNA segment, containing a replicon functional in E. coli,
b) a second DNA segment, containing the DNA fragment coding for the mobilisation function (mob site containing the oriT),
c) a third DNA segment, which recombines homologously in gram-positive bacteria and/or contains a replicon functional in Coryneform bacteria,
d) a DNA segment containing the sacB gene from Bacillus subtilis.

3. Vector according to claim 2,
characterised in that the vector pWJ 5 (trapping vector) is used, which vector is described by the restriction map reproduced in Figure 3 and consists of 11790 bp.

4. Insertion element of the type ISCg1 from C. glutamicum, C. fascians, C. herculis and B. flavum, consisting of approximately 1.45 kb and having the inversely repetitive ends of approximately 24 bp in length:

IR-L G G C c C T T C C g G T T T g G g G T a C A T c a

IR-R G G C t C T T C C t G T T T a G a G T g C A T t g

5. Insertion element according to claim 4,
characterised in that it consists of 1452 bp and possesses the base sequence:

GGCCCTTCGGTTTTGGGTACATCACAGAACCTGGCTAGCGGTGTAGACCCGAAAATA
10 20 30 40 50 60

AACGAGCCTTTTGTCAGGGTTAAGGTTTAGGTATCTAAGCTAACCAAACACCAACAAAAG
70 80 90 100 110 120

M K S T G N I I A D T I C R T - E
GCTCTACCCATGAAGTCTACCGGCAACATCATCGCTGACACCATCTGCCGCACTNGCGAA
130 140 150 160 170 180

L G L T I T G A S D A G D Y T L I E A D
CTAGGACTCACCATCACCGGCGCTTCCGATGCAGGTGATTACACCCTGATCGAAGCAGAC
190 200 210 220 230 240

A L D Y T S T C P E C S Q P G V F R H H
GCACTCGACTACACCTCCACCTGCCCAGAATGCTCCCAACCTGGGGTGTTTCGTCATCAC
250 260 270 280 290 300

T H R M L I D L P I V G F P P N C L S V
F T H R R V S T K L F I R L
ACCCACCGGATGCTCATTGATTTACCCATCGTCGGGTTTCCACCAAACTGTTTATCCGTC
310 320 330 340 350 360

Y L A T A A P T P H V S K S I S K Q N *
P R Y R C T N P T C K Q K Y F Q A E L S
TACCTCGCTACCGCTGCACCAACCCCACATGTAAGCAAAAGTATTTCCAAGCAGAACTAA
370 380 390 400 410 420

C A D H G K K V T H R V T R W I L Q R L
GCTGCGCTGACCACGGTAAAAAGGTCACCCACCGGGTCACCCGCTGGATTTTACAACGCC
430 440 450 460 470 480

A I D R M S V H A T A K A L G L G W D L
TTGCTATTGACCGGATGAGTGTTCACGCAACCGCGAAAGCACTTGGGCTAGGGTGGGATT
490 500 510 520 530 540

T C Q L A L D M C R E L V Y N D P H H L
TAACCTGCCAACTAGCCCTCGATATGTGCCGTGAGCTGGTCTATAACGATCCTCACCATC
550 560 570 580 590 600

D G V Y V I G V D E H K W S H N R A K H
TTGATGGAGTGTATGTCATTGGGGTGGATGAGCATAAGTGGTCACATAATAGGGCTAAGC
610 620 630 640 650 660

G D G F V T V I V D M T G H R Y D S R C
ATGGTGATGGGTTTGTCACCGTGATTGTCGATATGACCGGGCATCGGTATGACTCACGGT
670 680 690 700 710 720

P A R L L D V V P G R S A D A L R S W L
GTCCTGCCCGGTTATTAGATGTCGTCCCAGGTCGTAGTGCTGATGCTTTACGGTCCTGGC
730 740 750 760 770 780

G S R G E Q F R N Q I R I V S M D G F Q
TTGGCTCCCGCGGTGAACAGTTCCGCAATCAGATACGGATCGTGTCCATGGATGGATTCC
790 800 810 820 830 840

G Y A T A S K E L I P S A R R V M D P F
AAGGCTACGCCACAGCAAGTAAAGAACTCATTCCTTCTGCTCGTCGCGTGATGGATCCAT
850 860 870 880 890 900

H V V R L A G D K L T A C R Q R L Q R E
TCCATGTTGTGCGGCTTGCTGGTGACAAGCTCACCGCCTGCCGGCAACGCCTCCAGCGGG
910 920 930 940 950 960

K Y Q R R G L S Q D P L Y K N R K T L L
AGAAATACCAGCGTCGTGGTTTAAGCCAGGATCCGTTGTATAAAAACCGGAAGACCTTGT
970 980 990 1000 1010 1020

T T H K W L S P R Q Q E S L E Q L W A Y
TGACCACGCACAAGTGGTTGAGTCCTCGTCAGCAAGAAAGCTTGGAGCAGTTGTGGGCGT
1030 1040 1050 1060 1070 1080

D K D Y G A L K L A W L A Y Q A I I D C
ATGACAAAGACTACGGGGCGTTAAAGCTTGCGTGGCTTGCGTATCAGGCGATTATTGATT
1090 1100 1110 1120 1130 1140

Y Q M G N K R E A K K K M R T I I D Q L
GTTATCAGATGGGTAATAAGCGTGAAGCGAAGAAGAAAATGCGGACCATTATTGATCAGC
1150 1160 1170 1180 1190 1200

R V L K G P N K E L A Q L G R S L F K R
TTCGGGTGTTGAAGGGGCCGAATAAGGAACTCGCGCAGTTGGGTCGTAGTTTGTTTAAAC
1210 1220 1230 1240 1250 1260

L G D V L A Y F D V G V S N G P V E A I
GACTTGGTGATGTGTTGGCGTATTTCGATGTTGGTGTCTCCAACGGTCCGGTCGAAGCGA
1270 1280 1290 1300 1310 1320

N G R L E H L R G I A L G F R N L N H Y
TCAACGGACGGTTGGAGCATTTGCGTGGGATTGCTCTAGGTTTCCGTAATTTGAACCACT
1330 1340 1350 1360 1370 1380

I L R C L I H S G Q L V H K I N A L *
ACATTCTGCGGTGCCTTATCCATTCAGGGCAGTTGGTCCATAAGATCAATGCACTCTAAA
1390 1400 1410 1420 1430 1440

ACAGGAAGAGCC
1450

6. Insertion elements of the type ISBl1 from B. lactofermum consisting of approximately 1.45 kb and having inversely repetitive ends of approximately 26 bp in length:

IR-L G G C T C T T C C G T T t T T A G A G T G C A T T G

IR-R G G C T C T T C C G T T g T T A G A G T G C A T T G

7. Insertion elements of the type ISRF1 from C. fascians, consisting of approximately 1.3 kb and having inversely repetitive ends of approximately 18 bp in length:

IR-1 G G a C C t G A C C C C c A T t T G

IR-2 G G g C C c G A C C C C g A T a T G

8. Use of the insertion elements according to claims 5 to 7 for the mutagenesis of coryneform bacteria,
characterised in that
8.1 a vector which is mobilisable from an E. coli mobiliser strain and is not auto-transferable is constructed, which vector is composed of
8.1.1 a DNA segment, containing a replicon functional in E. coli,
8.1.2 a second DNA segment, containing the DNA fragment coding for the mobilisation function (mob site containing the oriT),
8.1.3 a third DNA segment, which recombines homologously in gram-positive bacteria and/or contains a replicon functional in Coryneform bacteria, and
8.1.4 a DNA segment containing the IS element,
8.2 this vector is transferred by conjugative transfer into the Coryneform recipient strain and
8.3 the transconjugant containing the vector is cultivated in the desired nutrient medium.

## Revendications

1. Procédé de mise en évidence d'éléments d'insertion (éléments IS) ou de transposons dans des bactéries coryneformes, qui comprend :
1.1. la construction d'un vecteur non autotransférable, mobilisable à partir d'une souche mobilisatrice E. coli, constitué :
1.1.1. d'un segment d'ADN, contenant un réplicon fonctionnel dans E. coli,
1.1.2. d'un deuxième segment d'ADN, contenant le fragment d'ADN codant pour la fonction de mobilisation (site Mob contenant l'oriT),
1.1.3. d'un troisième segment d'ADN, qui se recombine de manière homologue dans des bactéries Gram-positives et/ou contient un réplicon fonctionnel dans des bactéries coryneformes,
1.1.4. d'un segment d'ADN contenant le gène sacB, provenant du Bacillus subtilis,
1.2. la transmission de ce vecteur par transfert conjugué dans les souches de receveurs coryneformes,
1.3. la culture des transconjugants contenant le vecteur dans un milieu nutritif contenant 10 % de saccharose,
1.4. la lyse du clone résistant à la saccharose, la dissociation des plasmides par des endonucléases de restriction et l'analyse des fragments.

2. Vecteur mobilisable non autotransférable pour la mise en évidence d'éléments d'insertion ou de transposons dans des bactéries coryneformes, qui est composé :
a) d'un segment d'ADN contenant un réplicon fonctionnel dans E. coli,
b) d'un deuxième segment d'ADN contenant le fragment d'ADN codant pour la fonction de mobilisation (site Mob contenant l'oriT),
c) d'un troisième segment d'ADN qui se combine de manière homologue dans des bactéries Gram-positives et/ou contient un réplicon fonctionnel dans des bactéries coryneformes,
d) d'un segment d'ADN contenant le gène sacB provenant du Bacillus subtilis.

3. Vecteur selon la revendication 2,
caractérisé en ce que l'on utilise le vecteur pWJ5 (vecteur de départ), qui est décrit dans la carte de restriction reproduite dans la Fig. 3 et est constitué de 11790 bp.

4. Elément d'insertion du type ISCg1 provenant des souches C. glutamicum, C. fascians, C. herculis et B. flavum, constitué d'environ 1,45 kb et avec des extrémités à récurrence inverse d'une longueur d'environ 24 bp :

IR-L G G C c C T T C C g G T T T g G g G T a C A T c a
IR-R G G C t C T T C C t G T T T T a G a G T g C A T t g

5. Elément d'insertion selon la revendication 4, caractérisé en ce qu'il est constitué de 1452 bp et possède la séquence de base :

GGCCCTTCCGGTTTTGGGGTACATCACAGAACCTGGGCTAGCGGTGTAGACCCGAAAATA
10 20 30 40 50 60

AACGAGCCTTTTGTCAGGGTTAAGGTTTAGGTATCTAAGCTAACCAAACACCAACAAAAG
70 80 90 100 110 120

M K S T G N I I A D T I C R T - E
GCTCTACCCATGAAGTCTACCGGCAACATCATCGCTGACACCATCTGCCGCACTNGCGAA
130 140 150 160 170 180

L G L T I T G A S D A G D Y T L I E A D
CTAGGACTCACCATCACCGGCGCTTCCGATGCAGGTGATTACACCCTGATCGAAGCAGAC
190 200 210 220 230 240

A L D Y T S T C P E C S Q P G V F R H H
GCACTCGACTACACCTCCACCTGCCCAGAATGCTCCCAACCTGGGGTGTTTCGTCATCAC
250 260 270 280 290 300

T H R M L I D L P I V G F P P N C L S V
F T H R R V S T K L F I R L
ACCCACCGGATGCTCATTGATTTACCCATCGTCGGTTTCCACCAAACTGTTTATCCGTC
310 320 330 340 350 360

Y L A T A A P T P H V S K S I S K Q N *
P R Y R C T N P T C K Q K Y F Q A E L S
TACCTCGCTACCGCTGCACCAACCCCACATGTAAGCAAAAGTATTTCCAAGCAGAACTAA
370 380 390 400 410 420

C A D H G K K V T H R V T R W I L Q R L
GCTGCGCTGACCACGGTAAAAAGGTCACCCACCGGGTCACCCGCTGGATTTTACAACGCC
430 440 450 460 470 480

A I D R M S V H A T A K A L G L G W D L
TTGCTATTGACCGGATGAGTGTTCACGCAACCGCGAAAGCACTTGGGCTAGGGTGGGATT
490 500 510 520 530 540

T C Q L A L D M C R E L V Y N D P H H L
TAACCTGCCAACTAGCCCTCGATATGTGCCGTGAGCTGGTCTATAACGATCCTCACCATC
550 560 570 580 590 600

D G V Y V I G V D E H K W S H N R A K H
TTGATGGAGTGTATGTCATTGGGGTGGATGAGCATAAGTGGTCACATAATAGGGCTAAGC
610 620 630 640 650 660

G D G F V T V I V D M T G H R Y D S R C
ATGGTGATGGGTTTGTCACCGTGATTGTCGATATGACCGGGCATCGGTATGACTCACGGT
670 680 690 700 710 720

P A R L L D V V P G R S A D A L R S W L
GTCCTGCCCGGTTATTAGATGTCGTCCCAGGTCGTAGTGCTGATGCTTTACGGTCCTGGC
730 740 750 760 770 780

G S R G E Q F R N Q I R I V S M D G F Q
TTGGCTCCCGCGGTGAACAGTTCCGCAATCAGATACGGATCGTGTCCATGGATGGATTCC
790 800 810 820 830 840

G Y A T A S K E L I P S A R R V M D P F
AAGGCTACGCCACAGCAAGTAAAGAACTCATTCCTTCTGCTCGTCGCGTGATGGATCCAT
850 860 870 880 890 900

H V V R L A G D K L T A C R Q R L Q R E
TCCATGTTGTGCGGCTTGCTGGTGACAAGCTCACCGCCTGCCGGCAACGCCTCCAGCGGG
910 920 930 940 950 960

K Y Q R R G L S Q D P L Y K N R K T L L
AGAAATACCAGCGTCGTGGTTTAAGCCAGGATCCGTTGTATAAAAACCGGAAGACCTTGT
970 980 990 1000 1010 1020

T T H K W L S P R Q Q E S L E Q L W A Y
TGACCACGCACAAGTGGTTGAGTCCTCGTCAGCAAGAAAGCTTGGAGCAGTTGTGGGCGT
1030 1040 1050 1060 1070 1080

D K D Y G A L K L A W L A Y Q A I I D C
ATGACAAAGACTACGGGGCGTTAAAGCTTGCGTGGCTTGCGTATCAGGCGATTATTGATT
1090 1100 1110 1120 1130 1140

Y Q M G N K R E A K K K M R T I I D Q L
GTTATCAGATGGGTAATAAGCGTGAAGCGAAGAAAAAATGCGGACCATTATTGATCAGC
1150 1160 1170 1180 1190 1200

R V L K G P N K E L A Q L G R S L F K R
TTCGGGTGTTGAAGGGGCCGAATAAGGAACTCGCGCAGTTGGGTCGTAGTTTGTTTAAAC
1210 1220 1230 1240 1250 1260

L G D V L A Y F D V G V S N G P V E A I
GACTTGGTGATGTGTTGGCGTATTTCGATGTTGGTGTCTCCAACGGTCCGGTCGAAGCGA
1270 1280 1290 1300 1310 1320

N G R L E H L R G I A L G F R N L N H Y
TCAACGGACGGTTGGAGCATTTGCGTGGATTGCTCTAGGTTTCCGTAATTTGAACCACT
1330 1340 1350 1360 1370 1380

I L R C L I H S G Q L V H K I N A L *
ACATTCTGCGGTGCCTTATCCATTCAGGGCAGTTGGTCCATAAGATCAATGCACTCTAAA
1390 1400 1410 1420 1430 1440

ACAGGAAGAGCC
1450

6. Elément d'insertion du type ISB11 provenant de la souche B. lactofermentum et constitué d'environ 1,45 kb avec des extrémités à récurrence inverse d'une longueur d'environ 26 bp :

IR-L G G C T C T T C C G T T t T T A G A G T G C A T T G
IR-R G G C T C T T C C G T T g T T A G A G T G C A T T G

7. Elément d'insertion du type ISRF1 provenant de la souche C. fascians, constitué d'environ 1,3 kb et avec des extrémités à récurrence inverse d'une longueur d'environ 18 bp :

IR-1 G G a C C t G A C C C C c A T t T G
IR-2 G G g C C c G A C C C C g A T a T G

8. Utilisation de l'élément d'insertion selon les revendications 5 à 7 pour la mutagenèse de bactéries coryneformes, caractérisée en ce que :
8.1. on construit un vecteur non autotransférable, mobilisable à partir d'une souche mobilisatrice de E. coli, constitué :
8.1.1. d'un segment d'ADN, contenant un réplicon fonctionnel de E. coli,
8.1.2. d'un deuxième segment d'ADN, contenant des fragments d'ADN codant pour la fonction mobilisatrice (site Mob contenant l'oriT),
8.1.3. d'un troisième segment d'ADN, qui se recombine de manière homologue dans des bactéries Gram-positives et/ou contient un réplicon fonctionnel dans des bactéries coryneformes, et
8.1.4. d'un segment d'ADN contenant l'élément IS,
8.2. on transmet ce vecteur par transfert conjugué dans la souche de receveur coryneforme, et
8.3. on cultive le transconjugant contenant le vecteur dans le milieu nutritif souhaité.
